# EUROPEAN PATENT APPLICATION

(11) **EP 4 213 251 A1**
(43) Date of publication of application: **19.07.2023**
(21) Application number: 21866146.0
(22) Date of filing: 14.09.2021
(51) Int. Cl.: H01M 6/04, C07C 211/62

(54) **SUPRAMOLECULAR FLUID**

(30) Priority: 14.09.2020 ES 202030929
(71) Applicant: Universidade de Santiago de Compostela, 15782 Santiago de Compostela (ES)
(72) Inventor: GIMENEZ LOPEZ, Maria del Carmen, 15782 Santiago de Compostela (ES); RIVADULLA FERNANDEZ, Jose Francisco, 15782 Santiago de Compostela (ES); HERREROS LUCAS, Carlos, 15782 Santiago de Compostela (ES); LOPEZ BUENO, Carlos, 15782 Santiago de Compostela (ES)
(74) Representative: Patentree
(86) International application number: PCT/ES2021/070659
(87) International publication number: WO 2022/053735

(57) **Abstract**

The present invention relates to a supramolecular fluid for use in redox reactions. More specifically, the fluid of the invention comprises a quaternary ammonium salt. The invention also relates to compositions of the quaternary ammonium salt and to the uses of same.

## Description

### Technical Field

The present invention relates to a supramolecular fluid. More specifically, the fluid of the invention comprises a quaternary ammonium salt or a quaternary phosphonium salt. The invention also relates to compositions of the quaternary ammonium salt or of the quaternary phosphonium salt, and to the uses of same.

### Background of the Invention

Among the commonly used electrolytes, the bromide anion presents several advantages: it is inexpensive, has a high water solubility and a high diffusion coefficient. However, it causes undesired side reactions because it reacts with the bromine that is formed in the oxidation process, producing the tribromide ion (Br⁻₃) which precipitates as a yellow solid (Energy Environ. Sci, 2014, 7, 1990; Not. Commun. 6:7818 (2015*)*)*.*

In this way, it is still necessary to find an electrolyte with all the advantages of the bromide and that also avoids those side reactions in which the tribromide species is formed.

Among the metallic electrodes used, those based on transition metals such as copper present important advantages in the implementation of electrochemical processes, due to their low cost and availability in comparison with noble metals. However, they suffer from limited stability in aqueous electrolytes and especially those with chloride ions, due to the undesired formation of a solid film of copper chloride (CuCI) on the electrode surface, which reacts with more chloride ions to produce soluble anionic species (CuCl₂⁻) (Nature Communications 2018, 9, 1; Corrosion Science 2018, 140, 111).

Therefore, the identification of electrolyte media that inhibit copper corrosion in aqueous media with a high concentration of chloride ions (the case of seawater), where soluble charged species are formed (CuCl₂⁻), is still necessary.

### Brief Description of the Invention

The present invention provides a new fluid that is useful as an electrolyte in batteries (or storage cells). Furthermore, the authors have shown that this fluid inhibits or prevents electrode deterioration, for example electrode oxidation or electrode corrosion.

Thus, in a first aspect the invention refers to an electrolyte stable under atmospheric conditions characterised in that it comprises a quaternary ammonium halide or a quaternary phosphonium halide, with a concentration equal to or more than 0.6 m (0.6 molal) in an aqueous solution.

Furthermore, the authors of the invention found that the electrolyte of the invention is also capable of preventing the formation of tribromide and allowing the electrochemical reaction using bromide as electrolyte to be reversible. In particular, they found that a quaternary ammonium halide or a quaternary phosphonium halide, in a certain concentration range, completely prevents the formation of the tribromide ion.

In a second aspect, the invention refers to a battery (or storage cell) comprising the electrolyte of the invention.

A third aspect of the invention refers to the use of the electrolyte.

### Description of the drawings

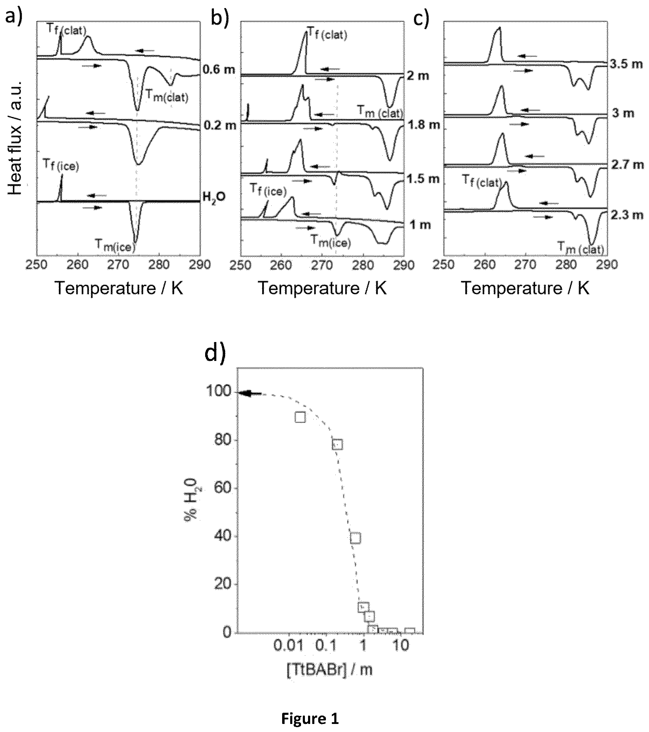

Figure 1 represents the heating and cooling curves of differential scanning calorimetry for various aqueous solutions of tetrabutylammonium bromide in water. It can be observed how the ice melting peak (at zero degrees Celsius) gradually disappears when the concentration of the ammonium salt is increased, until it disappears completely at 2 m. At this concentration and higher concentrations, only the characteristic double peak of clathrate melting is visible, demonstrating the absence of free water in the system.

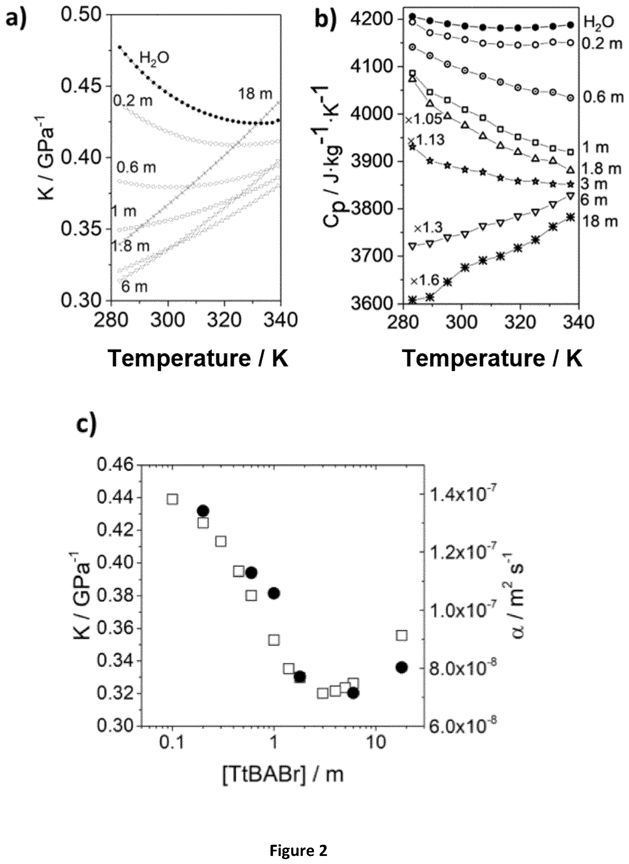

Figure 2 represents the variation of the compressibility of tetrabutylammonium bromide in water at different concentrations versus temperature (Figure 2a); the heat capacity of tetrabutylammonium bromide in water at different concentrations versus temperature (Figure 2b); and the compressibility and thermal diffusivity versus concentration of tetrabutylammonium bromide in water (molal) (Figure 2c) at room temperature. The white squares refer to compressibility and the black circles refer to thermal diffusivity. A minimum in compressibility and thermal diffusivity at room temperature is observed for the 2-4 m concentration range.

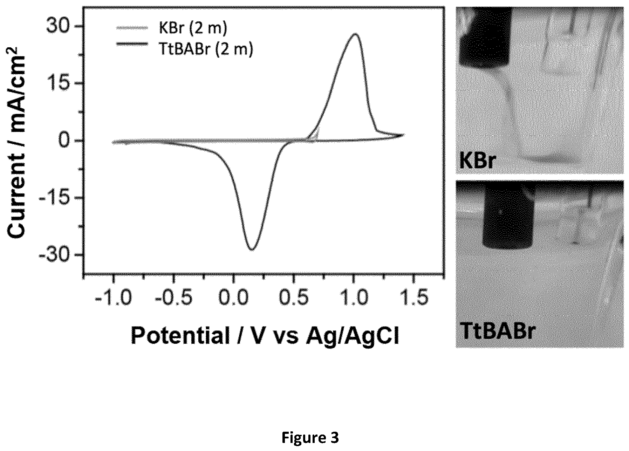

Figure 3 represents the cyclic voltammogram that occurs when tetrabutylammonium bromide at a concentration of 2 m is used as electrolyte and shows the comparison with the same redox reaction when potassium bromide 2 m is used.

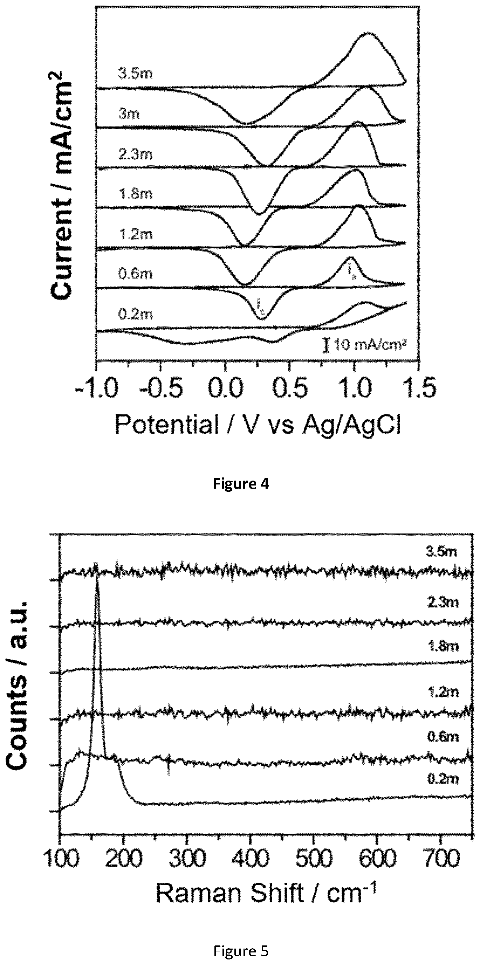

Figure 4 represents the cyclic voltammogram at different concentrations of tetrabutylammonium bromide in water. It can be observed that only at concentrations higher than 0.6 m the bromide redox process is reversible, based on the presence of well-defined anodic (iₐ) and cathodic (i_{c}) peaks, with a ratio close to 1. The current increases with the concentration of Br⁻ anions.
Figure 5 represents the Raman spectra of the electrodes after the redox reaction using different concentrations of tetrabutylammonium bromide as electrolyte. It is shown that the tribromide anion (band at 160 cm⁻¹) is formed only on the electrodes that were exposed to concentrations lower than 0.6 m, as a consequence of the low reversibility of the process.

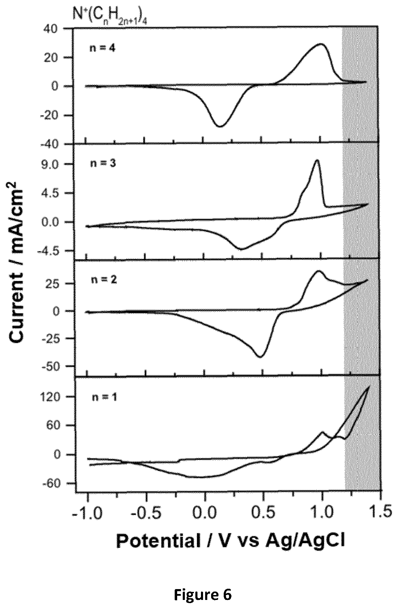

Figure 6 represents the cyclic voltammograms of different quaternary ammonium bromides.

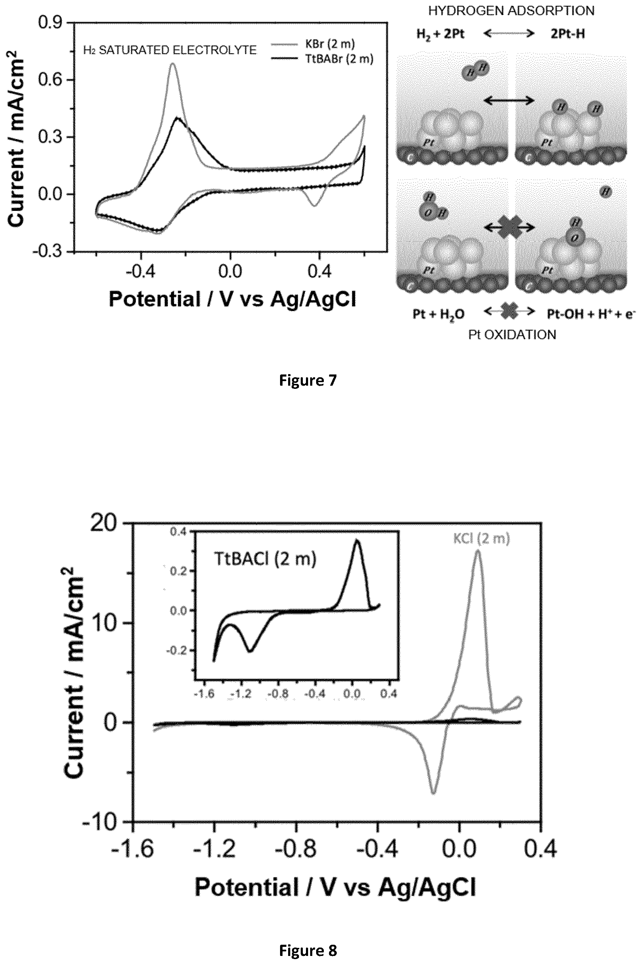

Figure 7 represents the cyclic voltammograms of Pt / C in a hydrogen saturated solution of 2 m KBr and 2 m TtBABr at 50 mV/s. The region between ≈ -0.6 to 0 V corresponds to the adsorption / desorption of hydrogen on the surface of platinum nanoparticles, while that between ≈ 0.2 to 0.6 V is related to the surface oxidation / reduction of Pt. The diagrams (right panel) depict these processes; the surface oxidation/reduction of Pt in water is inhibited in TtBA 2 m due to the low reactivity of water molecules in our supramolecular fluid. As shown in the figure, the cathodic peak at 0.38 V corresponds to the reduction of the electrochemically oxidized Pt surface. Interestingly, the oxidation of Pt in H₂O is completely inhibited in the aqueous TtBABr (2 m) solution, as reflected by the total absence of any measurable faradaic current at positive potentials. Note that the TtBA salt is not blocking the electrode surface, as demonstrated by the fact that Pt is still electrochemically active at negative potentials where hydrogen adsorption/desorption takes place. Therefore, the reactivity of the water molecules is limited by their strong interaction in the supramolecular fluid structure.
Figure 8 represents the cyclic voltammograms for a copper electrode (wire) used as working electrode, comparing the corrosion inhibiting effect of the tetrabutylammonium chloride (2 m) electrolyte with the corrosive effect of a potassium chloride solution, both solutions having the same aqueous concentration of chloride ions (2 m) in a nitrogen atmosphere and at a rate of 50 mV/s.

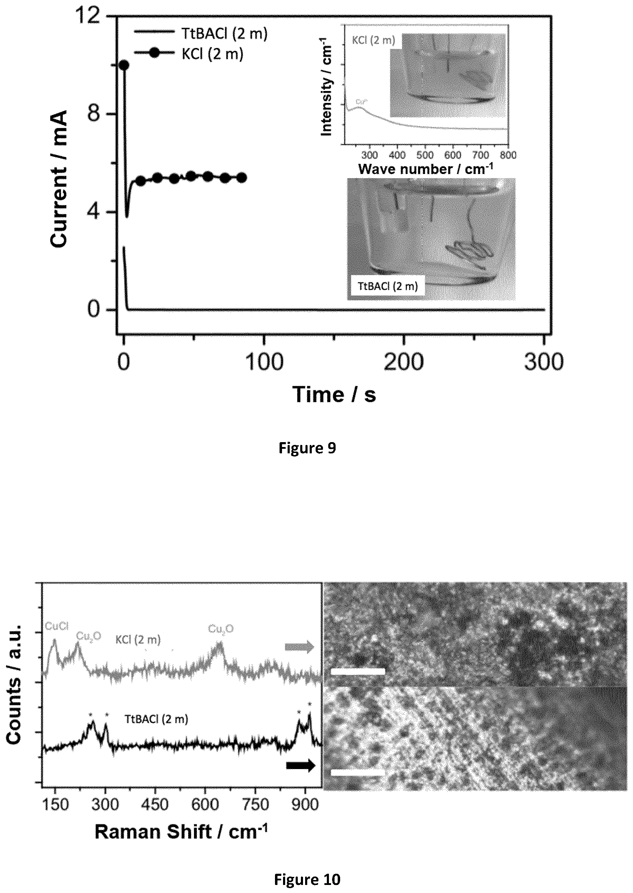

Figure 9 represents a picture of the electrochemical cell after chronoamperometric experiments (0.3 V) using tetrabutylammonium chloride (2 m) as electrolyte (colourless solution). After the chronoamperometric experiment, following the application of 0.3 V for 300 seconds, no change in the colour of the electrolyte is observed. However, when potassium chloride (2 m) is used as electrolyte instead of tetrabutylammonium chloride, the initially colourless potassium solution turns brown due to copper corrosion, which is in agreement with the presence of a band below 300 nm in the ultraviolet-visible spectrum of the same.
Figure 10 represents Raman spectra of the surface of a copper electrode (wire) used as working electrode, comparing the corrosion inhibiting effect of the tetrabutylammonium chloride (2 m) electrolyte with the corrosive effect of a potassium chloride solution at the same concentration (2 m). The electrode surface is completely intact after chronoamperometric experiments (0.3 V) in the presence of TtBACI (only bands related to the presence of the TtBA⁺ cation are observed, *), while in potassium chloride (2 m) the presence of copper oxide and copper chloride is observed as a consequence of the corrosion. The images on the right show the analysed areas (scale: 30 µm).

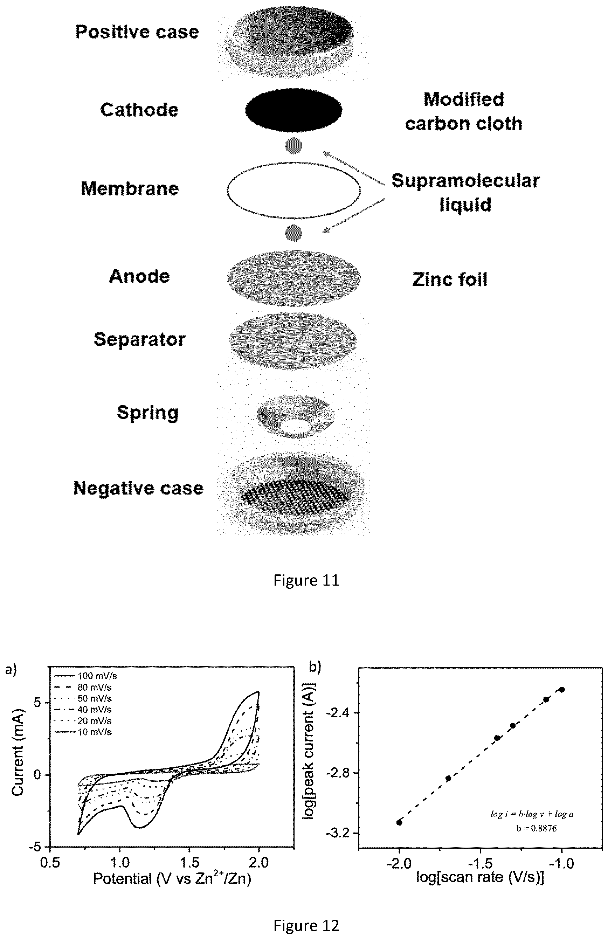

Figure 11 schematically represents a Zn-Br button storage cell using the supramolecular fluid as electrolyte doped with an electroactive inorganic salt (ZnSO₄) and tetramethyl orthosilicate, and modified carbon cloth as cathode.
Figure 12 shows the electrochemical behaviour of the supramolecular fluid doped with ZnSO₄ in the configuration indicated in example 7.3. (a) Cyclic voltammogram at different scan rates showing the reversibility of the process (Br/Br₂). (b) Plot of the variation of log (current (A)) with log (scan rate (mV/s)) of the anodic peak. The value of *b* = 0.8876 obtained using the equation *log i = b·log υ + log a* indicates that the electrochemical kinetics is controlled by a surface redox process and without intercalation (V. Augustyn et al. Nature Materials 2013, 12, 518).

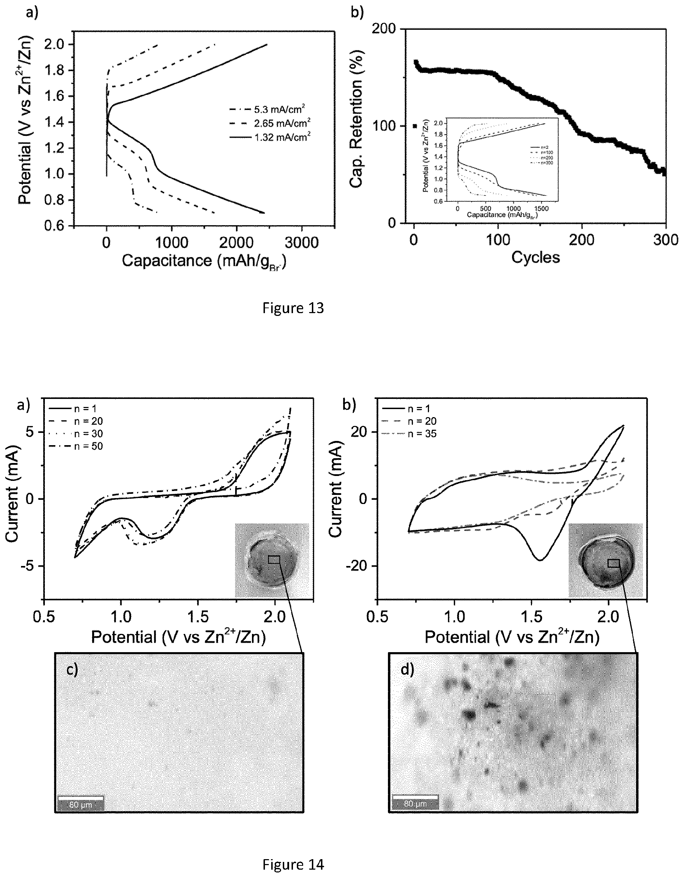

Figure 13 shows the electrochemical behaviour of the supramolecular fluid doped with ZnSO₄ and tetramethyl orthosilicate in the configuration shown in example 7.3. (a) Charge-discharge cycles at different current densities (5.3, 2.65 and 1.32 mA/cm²). Reversible oxidation/reduction of bromide to bromine ions occurs at 1.9 and 1.1 V, respectively. The specific capacitances were normalised with respect to the mass of the oxidized Br-. (b) Variation of the capacitance retention (%) after 300 cycles. The inserted image shows the profile of the charge/discharge cycles at 2.65mA/cm², for cycle 2, 100, 200 and 300.
Figure 14 shows the cyclic voltammetry after several cycles (a) of the supramolecular fluid doped with ZnSO₄ in the configuration of example 7.3 and (b) of an aqueous ZnSO₄ solution (without the supramolecular fluid), both measured in the same configuration of example 7.3. The images inserted in the graphs are pictures of the membrane separator after the stability tests. In the experiment without supramolecular fluid (d), yellow coloured areas are observed on the surface of the separator (indicative of the presence of tribromide, Br₃⁻), while in the experiment with the supramolecular fluid (c) the separator is free from this colouring.

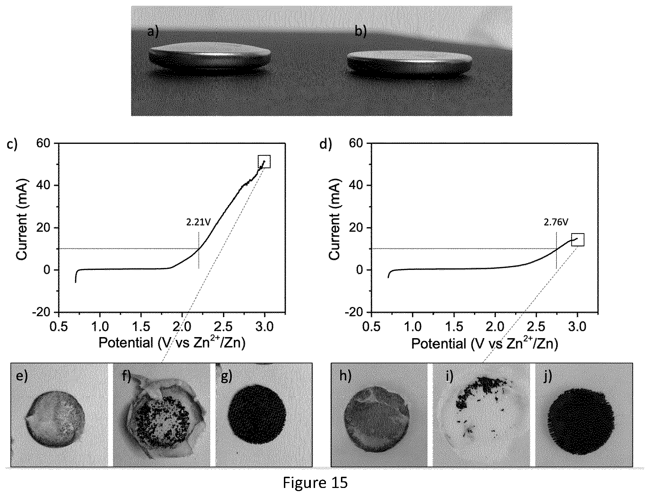

Figure 15 is a photo of a button cell assembled in the absence of the supramolecular fluid (a) and in the presence of the supramolecular fluid doped with ZnSO₄ (b), according to the diagram of example 7.3, highlighting the volume changes produced in the sealed cases after increasing the potential up to 3 V at 10 mV/s (potential window: +0.7 V / +3V) (c, d). Photos of the zinc anode (e, h), membrane separator (f, i) and modified carbon cloth (cathode) (g, j) of disassembled button storage cells, after increasing the positive potential window from 0.7 V to 3 V at 10 mV/s in the absence and presence of the supramolecular fluid, respectively.

### Detailed description of the invention

The present invention provides a new electrolyte which is formed by a supramolecular fluid with the physicochemical characteristics that are detailed in the present specification and characterise said fluid.

A first aspect of the invention refers to an electrolyte stable at atmospheric conditions characterised in that it comprises a quaternary ammonium halide or a quaternary phosphonium halide, with a concentration equal to or more than 0.6 m in an aqueous solution. In a particular embodiment, the concentration of the quaternary ammonium halide or a quaternary phosphonium halide is equal to or more than 2 m. In another particular embodiment, the concentration of the quaternary ammonium halide or a quaternary phosphonium halide is comprised between 0.6 m and 30 m.

In this way, at a concentration of 2 m or higher, each macromolecule of this fluid is composed of a quaternary ammonium halide or a quaternary phosphonium halide and a number of water molecules between 30 and 38, so that all water molecules are part of those structures, without any free water remaining. Therefore, the entire fluid is supramolecular.

In the present invention, "supramolecular fluid" means a fluid composed of the association of water molecules, with a stable structure, such that all water molecules are part of its structure and behave collectively in terms of their physicochemical and transport properties.

The fluid of the present invention is stable under atmospheric conditions. Atmospheric conditions are considered to be laboratory standard working conditions in which the ambient temperature and pressure do not change; in general, atmospheric conditions can be between 15°C and 30°C, at a pressure of approximately 1 atm. The fluid of the invention is stable under these conditions, and in fact it is possible to obtain it at atmospheric conditions, without needing any variation in either temperature or pressure to form it, unlike clathrates which need low temperatures and high pressures to form their crystalline structures. The fluid of the invention, once formed, is stable and for this reason its properties do not vary, nor does it decompose over time, and studies carried out by the authors of the invention demonstrate that it is stable for more than 6 months.

The formation of the fluid of the invention depends on the concentration of the ammonium or phosphonium halide in aqueous solution. In a particular embodiment, the aqueous solution is water. It can thus be observed that at concentrations below 2 m, part of the water in the solution is free, and the lower the concentration of the ammonium salt, the greater the amount of free water. At a concentration equal to or more than 2 m, there is no free water present and all the water molecules form a structural part of the fluid (Figure 1, example 1).

In a preferred embodiment, of the first aspect of the invention, the concentration of the quaternary ammonium salt or the quaternary phosphonium salt is between 2 m and 30 m, preferably between 2 m and 10 m, more preferably between 2 m and 4 m, preferably between 2.3 m and 10 m, preferably between 2.3 m and 3.6 m, preferably between 2 m and 3.6 m.

As can be observed also in Figure 1, the melting point temperature of the fluid of the invention (i.e. with concentrations above 2 m) is 9-12°C, therefore higher than the melting point of ice, indicating a higher structural stability.

The thermal conductivity of the fluid of the invention also demonstrates that the concentration of the ammonium or phosphonium is essential and it can be observed that as the concentration increases, the thermal diffusivity decreases (Figure 2). In addition, other physicochemical properties of the fluid, such as its compressibility, speed of sound propagation, or heat capacity, show an anomaly at a concentration of 2 m or higher, demonstrating the uniqueness of the fluid in those compositions.

In examples 1 and 2 of the present specification, it is demonstrated that the fluid of the invention is a supramolecular fluid, with characteristic properties thereof, in which the water and ammonium salt come together to form a unique structure, which can resemble that of a crystalline clathrate in solid state; with the advantage that our fluid has no such crystalline structure nor is it a solid and therefore it is possible to use it in applications that a clathrate would not be able to, such as, for example, as an electrolyte in a redox process.

In example 3A, it is shown how the fluid of the invention is useful as an electrolyte in a Br₂ / Br⁻ redox process. In this example, the efficacy of quaternary ammonium bromide at a concentration of 2 m is compared with potassium bromide, both employed as electrolytes, and it is demonstrated that while when using potassium bromide the tribromide species (Br₃⁻) is formed and the redox reaction is less efficient or terminated, when quaternary ammonium bromide is used at a concentration of 2 m, this by-product is not formed (see example 3C and Figure 5). This is because the quaternary ammonium bromide fluid at a concentration of 2 m is able to capture Br₂ and thus prevent the formation of tribromide. An additional advantage of the fluid of the invention is that it allows the Br₂ confined within it to be reduced to bromide, favouring a reversible electrolytic process. Therefore, when the quaternary ammonium bromide fluid is used at a concentration of 2 m, the redox process is reversible.

As demonstrated in example 3B, at different concentrations of the fluid, for example at a concentration of 2 m and above, the same results are obtained, making the redox process reversible and avoiding the formation of tribromide. But even at lower concentrations the process is also reversible. Thus, a mixture of tetrabutylammonium bromide in water at lower concentrations, in which not all the water is forming a kind of clathrate as it occurs in the fluid, but some percentage of free water is observed (see Example 1 and Figure 1), is also useful to carry out a redox process and, moreover, to make this Br₂ / Br⁻ redox process reversible.

When ammonium bromide is used at concentrations below 0.5 m, the redox process is not reversible, as shown in Figure 4 at a concentration of 0.2 m.

In a preferred embodiment, the formula of the quaternary ammonium halide of the fluid of the invention is X⁻(Alq₄N)⁺, where X is selected from chlorine, bromine and iodine, and Alq is a linear or branched C₁-C₄ alkyl. In another preferred embodiment, the formula of the quaternary phosphonium halide of the fluid of the invention is X⁻(Alq₄P)⁺, where X is selected from chlorine, bromine and iodine, and Alq is a linear or branched C₁-C₄ alkyl. In a particular embodiment, Alq is selected from methyl, ethyl, propyl, butyl. In a particular embodiment, the quaternary ammonium halide used in the present invention has the following formula: [CₙH₂ₙ₊₁]₄N⁺Br⁻, where n is 1, 2, 3 or 4.

When the alkyl chain is butyl, a higher efficiency in the reversibility of the Br₂ / Br⁻ redox reaction has been observed (see example 4 and Figure 6). Thus, in a more preferred embodiment, the alkyl chain is butyl. In a particular embodiment, the ammonium employed in the fluid of the invention is tetrabutylammonium bromide hydrated with between 30 and 38 molecules of water.

In a particular embodiment, the electrolyte consists of the fluid as described above.

In a second aspect, the invention refers to a battery (or storage cell) comprising the electrode of the first aspect of the invention, and at least one electrode.

The electrode can be selected by the skilled person in the art according to his needs, based on general knowledge of the art. For example, these can be metallic electrodes, such as copper or platinum electrodes, or carbon electrodes such as modified glassy carbon or carbon cloth.

It is possible to use the fluid of the invention doped with an electroactive inorganic salt, for example an inorganic salt in which the cation is Zinc, and additionally it is also possible to add other substances, for example tetramethyl orthosilicate, which improves durability. In a particular embodiment, the electrolyte of the invention is doped with an electroactive inorganic salt.

In the present invention, the term "doping or doped" refers to the fluid containing a certain amount of another substance or several substances, more specifically, it contains a total amount of substances of at least 5% by weight relative to the weight of the fluid, preferably between 5% and 50% by weight relative to the weight of the fluid.

The term "electroactive inorganic salt" in the present invention refers to inorganic salts which enable, promote or participate in electrolytic processes, for example salts having a zinc, lithium, sodium, potassium, magnesium or aluminium cation, which is reduced and oxidized on the surface of the anode (for example, the anode could be a zinc, lithium, sodium, potassium, magnesium and aluminium plate, respectively).

Doping the electrolyte of the invention with an electroactive inorganic salt has the advantage of allowing the development and construction of new reversible, stable and durable storage cells using low-cost cathodes (positive electrodes) made of commercially available carbon, for example zinc-bromide storage cells as shown in example 7. The storage cell of example 7 further shows several advantages of the electrolyte of the invention. In one particular case, contrary to existing Zn-ion storage cells, in the storage cells of the present invention the bromide ions do not require intercalation in synthetically complex and expensive cathodes. Furthermore, the storage cells of the present invention simultaneously solve two important limitations in the development of aqueous zinc storage cells: the formation of zinc dendrites at the anode (inhibiting effect of tetrabutylammonium) and the electrolysis of water (the clathrate structure reduces the reactivity of the water molecules). The flexibility in the use of the electrolyte of the invention further allows the construction of button-type storage cells.

Thus, in a particular embodiment, the invention refers to a battery or storage cell comprising the fluid of the invention and an electroactive inorganic salt. In a particular embodiment, the electrolyte of the invention has at least 5% of an electroactive inorganic salt relative to the weight of the fluid, as described above. In a particular embodiment, the electroactive inorganic salt is selected from a salt of Zinc, Lithium, Sodium, Potassium and Aluminium.

In a particular embodiment, the invention refers to a battery or storage cell wherein the electroactive inorganic salt has a cation selected from Zinc, Lithium, Sodium, Potassium and Aluminium and wherein the anode is selected from Zinc, Lithium, Sodium, Potassium and Aluminium, respectively.

In another particular embodiment, the invention refers to button-type storage cells.

In a third aspect, the invention refers to the use of the electrolyte of the first aspect of the invention in electrochemical reactions or in energy applications.

As demonstrated in example 3, the fluid of the invention is capable of encapsulating bromine and thus achieving a reversible process. Thus, the invention also refers to the use of the electrolyte of the first aspect of the invention for encapsulating substances. Preferably the substances are polar substances.

As shown in example 5, the fluid of the invention strongly interacts with water and thus prevents oxidation of the platinum electrode. In example 6, it is shown that the fluid of the invention can additionally act as an electrochemical medium inhibiting the corrosion of copper electrodes in water, in the presence of chloride ions. This new fluid prevents oxidation of the copper surface and its subsequent dissolution, as it restricts the mobility of both water molecules and chloride ions, preventing the formation of soluble charged species (CuCl₂⁻). Therefore, this new fluid would allow the exploitation of cathodic (reduction) processes in electrochemical cells, in which the stability of copper and alloy electrodes is essential.

Thus, in a particular embodiment, the invention relates to the use of the electrolyte of the invention to inhibit electrode deterioration, particularly in electrochemical cells. In a more particular embodiment, electrode deterioration is due to oxidation or corrosion.

The invention also relates to a process for the preparation of the fluid of the invention comprising mixing a quaternary ammonium salt or a quaternary phosphonium salt with water in quantities to obtain a concentration range equal to or more than 0.6 m.

To prepare the mixture it may be necessary to stir; the skilled person in the art knows different stirring methods that can be used in this process, depending on the quantities being prepared, for example magnetic stirring or mechanical stirring.

The following examples serve to illustrate the invention and are not in any case a limitation thereof.

### Materials and methods

Potassium bromide (KBr), potassium chloride (KCI), tetramethyl ammonium bromide (TMABr, 98%), tetraethyl ammonium bromide (TEABr, 98%), tetrapropyl ammonium bromide (TPABr, 99%), tetrabutylammonium chloride (TBACI, 98%), polyvinyl difluoride, zinc sulphate, orthosilicate tetramethyl and N-methylpyrrolidone (NMP) were purchased from Sigma. Tetrabutylammonium bromide (TtBABr, >98%) was purchased from TCI. Graphite nanoplatelets (2299 GNP) were purchased from Asbury. Pt nanoparticles adsorbed on carbon (Pt/C) (20%) were purchased from Johson Matthey. Solutions of the different ammonium and potassium salts were prepared by dissolving the particular salt in Mili-Q grade water. Carbon black Super P was purchased from Alfa Aesar. All chemicals were used without further purification. The Zn plate (0.1 mm thick, 99.95%) was purchased from Goodfellow, plain carbon cloth from FuelCellStore and Whatman glass filter paper from Sigma.

Electrochemical experiments were carried out on a computer-controlled potentiostat (Autolab 201A) at room temperature using a conventional three-electrode cell with a nitrogen or hydrogen saturated aqueous solution, depending on the experiment. A Pt wire was used as a counter electrode and a silver/silver chloride (Ag/AgCI) electrode as a reference electrode. A glassy carbon electrode (GCE, 3 mm diameter) modified with graphite nanoplatelets or Pt/C (20%) (Pt content 14 µg cm⁻²) was used as working electrode. Before use, the glassy carbon electrode was mechanically polished with aqueous suspensions of alumina powder (0.05 µm), rinsed with Mill-Q water and acetone and left to dry under nitrogen. For the experiments demonstrating the anti-corrosion properties of our supramolecular fluid, a copper wire was used as working electrode. Cyclic voltammograms were carried out in a solution of either a quaternary ammonium salt or a potassium salt at room temperature with a scan rate of 50 mV/s and chronoamperometry with the copper electrode, applying 0.3 V.

DSC measurements were carried out using a TA Instruments Q200 system. Adiabatic compressibility data were obtained from density and ultrasound velocity measurements (~ 3 MHz) recorded with an Anton Paar DSA 5000 calibrated with water and dry air. Specific heat was measured on a Setaram Micro DSC-III, also calibrated with water. Thermal conductivity measurements were performed at room temperature under ambient pressure, using the 3-omega method, in a configuration as described in C. López-Bueno, D. Bugallo, V. Leborán, F. Rivadulla, Phys. Chem. Chem. Phys. 2018, 20, 7277. A small amount of solution (1 µl) was used for each measurement, to avoid the effect of convective flows. All measurements were repeated at least three times, for reproducibility. Raman spectra were measured in solid state at ambient conditions with a Renishaw Raman spectrometer model InVia Reflex, using a laser wavelength of 514 nm. Samples were transferred from the electrode to a glass substrate for measurement. UV-vis spectra were performed in quartz cuvettes with a Perkin Elmer Lambda 25 UV/Vis spectrometer at ambient conditions.

### Example 1

A series of solutions were prepared in deionised water with different concentrations of TtBABr, from 0.2 m to 18 m. Calorimetric measurements (DSC) of these solutions show that the behaviour up to ≈0.6 m is similar to that of pure water, with an exothermic peak at the ice formation temperature and an endothermic peak at the 0°C melting temperature. At [TtBABr] ≥ 0.6 m, the ice peaks are progressively suppressed until they almost completely disappear at 1.8 m. At this concentration, exothermic/endothermic peaks characteristic of the formation/melting of the clathrate hydrate crystal lattice appear. The value obtained for the formation temperature is -8°C (265 K) and for the melting temperature is 9°C-12°C (282 K-286 K), and the value obtained for the enthalpy of fusion is ΔH_{melting} = 202 J/g. By comparing the area of the endothermic peaks at 0°C with that of pure water, the fraction of free water (available to form ice) in each solution is extracted. At 1.8 m, less than 1% of the water molecules are available to form ice, while no free water is detected in the 2 m solution or at concentrations higher than this (see Figure 1). Thus, calorimetric experiments confirm that the mixture of tetrabutylammonium bromide and water at concentrations equal to or more than 2 m is in a liquid state in which all water molecules are not free and participate in a structure similar or comparable to that of a solid clathrate hydrate at low temperature.

A similar behaviour with respect to the absence of free water to form ice, the similar variation in thermal conductivity, compressibility, heat capacity and thermal diffusivity was observed in tetrabutylammonium phosphonium solutions in a concentration range between 1-3 m.

### Example 2

A series of solutions with different concentrations of TtBABr, from 0.2 m to 18 m, were prepared in deionised water and their adiabatic compressibility (K) was studied and compared with that of pure water (see Figure 2a).

Thermal contraction makes most liquids less compressible as the temperature decreases. However, water shows minimal compressibility at ≈ 330 K, with a rapid increase below this temperature due to the formation of hydrogen bridges (L. B. Skinner, C. J. Benmore, J. C. Neuefeind, J. B. Parise, J. Chem. Phys 2014, 141, 214507). Considering that the water energy can be minimized through this hydrogen bridge lattice, the dependence of the adiabatic compressibility (K) on temperature is dominated by temperature and structural fluctuations, above and below the minimum value of the same, respectively (D. Schlesinger, K. T. Wikfeldt, L. B. Skinner, C. J. Benmore, A. Nilsson, L. G. M. M. Pettersson, The Journal of Chemical Physics 2016, 145, 084503). A minimum at ≈ 330 K is also observed in the heat capacity (Cp) of water because hydrogen bonds can store a significant amount of energy. Consequently, any long-range modification of the hydrogen bonding lattice of liquid water by hydrophobic solvation should be reflected in the magnitude and temperature dependence of K and Cp.

Studies show that the dissolution of TtBABr in water does indeed produce a rapid decrease in K and heat capacity Cp (see Figure 2b), and the suppression of the minimum in the temperature dependence of both quantities. In particular, the decrease in K at low temperatures suggests that the K-dominant structural fluctuations (T < Tmin) in water are progressively replaced by temperature fluctuations as [TtBABr] increases. Cp also decreases with increasing [TtBABr], reflecting the suppression of the contribution of hydrogen bridges to low-temperature energy storage. However, the negative dCp / dT coefficient up to ≈ 3 m shows that the translational and rotational modes (the main contributors to Cp above room temperature in liquid water) are largely suppressed at these TtBABr concentrations (at least up to 340 K). On the other hand, K increases again with increasing [TtBABr], and the characteristic variations of dK / dT> 0 and dCp / dT> 0 of molecular liquids are recovered. [T. S. Banipal, S. K. Garg, J. C. Ahluwalia, The Journal of Chemical Thermodynamics 1991, 23, 923]. Our studies demonstrate that both K (293 K) and dCp / dT (293 K) exhibit a minimum at [TtBABr] ≈ 1.4-2.2 m (see Figure 2c). The thermal diffusivity, α = κ / (ρCp) also shows a minimum in the same region as K. The extent and strength of hydrogen bridges is determinant in the energy transfer in liquid water, through the coupling of -OH excitations on several H₂O molecules. The reduction of α is much larger than expected based on purely local coordination and further supports the fact that hydrophobic solvation of TtBA⁺ modifies the structure of hydrogen bridges of water in the lattice, beyond the immediate molecular environment. Cooling the 1.8 m solution below the formation temperature of the solid clathrate TtBABr·32H₂O does not recover a high value of the thermal conductivity as in crystalline ice. This suggests poor long-range order, with orientation changes between supramolecular assemblies, or movements of the TtBA⁺ chains within the water boxes (or a combination of both). This situation resembles more a liquid-glass transition than a true crystallisation (as in pure ice, for example) where new and stronger long-range bonds are formed from a disordered liquid.

### Example 3

A) A comparison was made between the cyclic voltammograms (in a potential window between +1.0 and +1.4 V) obtained using TtBABr and KBr as electrolyte, both at the same concentration (2 m) at room temperature and with a scan rate of 50 mV/s, using as working electrode a glassy carbon electrode (GCE, 3 mm diameter) modified with graphite nanoplatelets. While in KBr the oxidation of Br⁻ to Br₂ is totally irreversible with the formation of the potassium salt of Br₃⁻ (yellow solid) which falls off the electrode to the bottom of the electrochemical cell, in TtBABr the oxidation is totally reversible (iₐ/i_{c}^{~}1; iₐ being the anodic current and i_{c} the cathodic current), allowing the potential window to be extended to 1.4 V in the absence of the formation of the yellow solid (KBr₃), see Figure 3. The use of tetrabutylammonium bromide as electrolyte allows extending the potential window from 0.7 to 1.4 V because the redox process is fully reversible.
B) Cyclic voltammograms (in a potential window between +1.0 and +1.4 V) of TtBABr solutions in water as electrolyte at different concentrations (from 0.2 m to 3.5 m) were performed at room temperature and with a scan rate of 50 mV/s, using as working electrode a glassy carbon electrode (GCE, 3 mm diameter) modified with graphite nanoplatelets. The values obtained are shown in Figure 4. The experiment demonstrates that the oxidation of Br⁻ to Br₂ is highly reversible for concentrations equal to or higher than 0.6 m, for which the anodic and cathodic value increases with the concentration and the ratio between them (iₐ/i_{c}) is practically 1.
C) RAMAN spectroscopy measurements (between 100 and 800 cm⁻¹ at a wavelength of 514 nm) were performed on the surface of each of the working electrodes after they were used in the voltammetry studies described in example 3B, using TtBABr as electrolyte at different concentrations (0.2 m-3.5 m). The spectra show the presence of a peak (160 cm⁻¹) of tribromomide (Br₃⁻) only at low concentrations of TtBABr (0.2 m). In this experiment, above a concentration of 0.6 m, the Br₃⁻ peak is no longer observed, because in TtBABr at high concentrations, the Br₂/Br⁻ redox process becomes reversible inhibiting the formation of tribromide (Br₃⁻), see Figure 5.

The tetrabutylammonium cation is able to inhibit at high concentrations (i.e. 0.6 m) the formation of the tribromide ion which is formed by the attack of a Br⁻ ion on the electrochemically formed Br₂.

### Example 4

Cyclic voltammograms (in a potential window between +1.0 and +1.4 V) were performed using as electrolyte solutions of different quaternary ammonium bromides with different chains (methyl (n=1), ethyl (n=2), propyl (n=3) and butyl (n=4)) in water at the same concentration (2 m) at room temperature and with a scan rate of 50 mV/s, using as working electrode a glassy carbon electrode (GCE, 3 mm diameter) modified with graphite nanoplatelets. It is observed that the anodic faradic current at positive potentials (> 1.2 V) associated with the formation of Br₃⁻ increases with decreasing number of carbons in the alkyl chain (see Figure 6). The comparison of these voltammograms demonstrates, therefore, that the reversibility of the oxidation of Br⁻ to Br₂ depends on the size of the alkyl chain, being maximum (iₐ/i_{c}~1; iₐ being the anodic current and i_{c} the cathodic current) for the butyl (n=4). And only the tetrabutylammonium cation is able to inhibit the formation of Br₃⁻ at high potentials (i.e. > 1.2, shaded area) while presenting the best reversibility (iₐ/i_{c}~1).

### Example 5

The oxidation of Pt (0) in water at positive potentials, i.e. Pt + H₂O → Pt-OH + H⁺ + e⁻ (and its reverse reduction reaction) is a well-known process [S. Gilman, Electrochimica Acta 1964, 9, 1025]. To test whether the supramolecular liquid structure restricts the participation of water molecules in the redox process occurring on a Pt surface, the cyclic voltammogram (in a potential window between -6.6 and +0.6 V) of Pt nanoparticles on carbon (Pt/C, 20%) supported on a glassy carbon electrode (GCE, 3 mm diameter) using as electrolyte an aqueous solution of TtBABr (2 m), with a scan rate of 50 mV/s at room temperature, was carried out and compared with that obtained using KBr as electrolyte under the same conditions, see Figure 7. The Pt nanoparticles exhibit the expected electrochemical activity in a hydrogen-saturated solution of KBr (2 m), i.e. H₂ molecules show reversible adsorption/desorption on the Pt surface at negative potentials (from ≈ -0.6 to 0 V), whereas H₂O molecules oxidize the Pt surface at a potential above ~0.4 V. The cathodic peak at 0.38 V in the KBr solution corresponds to the reduction of the electrochemically oxidized Pt surface. In contrast, the oxidation of Pt by H₂O is completely inhibited in the aqueous TtBABr solution (2 m), as reflected by the absence of any measurable faradic current at positive potentials. It is important to note that TtBA⁺ is not blocking the electrode surface, as evidenced by the fact that Pt remains electrochemically active at negative potentials, where hydrogen adsorption/desorption takes place. Therefore, it is the strong interaction between H₂O molecules in the compact structure of this supramolecular fluid that limits its reactivity.

### Example 6

A) The electrochemical applications of several non-noble metals such as Cu are severely limited by their stability in an aqueous environment, especially in the presence of chloride (Cl⁻) ions, due to corrosion [Y. Wang, B. Liu, X. Zhao, X. Zhang, Y. Miao, N. Yang, B. Yang, L. Zhang, W. Kuang, J. Li, E. Ma, Z. Shan, Nature Communications 2018, 9, 1; Y. Qiang, S. Fu, S. Zhang, S. Chen, X. Zou, Corrosion Science 2018, 140, 111]. To investigate whether our supramolecular fluid ([TtBACI] ≥ 2 m) can reduce the corrosion of such metals when acting as an electrolyte, the electrochemical properties of a copper wire (working electrode) in a three-electrode configuration using Ag/AgCI as reference electrode and a Pt wire as counter electrode were investigated and compared with those obtained for an aqueous KCI solution at the same concentration and conditions (nitrogen atmosphere, room temperature and scan rate of 50 mV/s). The corrosion of Cu is chemically complex and, besides the oxidation to copper oxides (Cu₂O), an additional two-step process is known in the presence of Cl⁻. In this case, a CuCl film grows on the electrode surface, according to A. R. Langley, M. Carta, R. Malpass-Evans, N. B. McKeown, J. H. P. Dawes, E. Murphy, F. Marken, Electrochimica Acta 2018, 260, 348.

   Cu (s) + Cl⁻ (aq) ⇔ CuCl (s) + e⁻

   and subsequently forming a soluble chloro-cuprate (I) species:

   CuCI (s) + Cl⁻ ⇔ CuCl₂⁻ (aq)

   The cyclic voltammogram of a Cu wire obtained in a KCI solution (2 m) shows that Cu corrosion takes place through a redox process that involves the oxidation of Cu(0) to Cu(I) at a potential of ~0 V with a separation between the anodic and cathodic peak of ~0.216 V (ΔE = Ea-Ec), see Figure 8. The low coulombic efficiency of the process (ratio between the anodic and cathodic peak much higher than 1, (iₐ/i_{c}) ≈ 2.42) confirms that part of the Cu atoms are irreversibly oxidized to CuCl₂⁻ (aq) which is lost away from the electrode surface, additionally forming Cu₂O in water which is deposited on the electrode. The formation and loss of CuCl₂⁻ (aq) is in agreement with the fact that at a potential above 0.2 V the current remains positive, indicating that new Cu atoms are continuously appearing on the electrode surface and oxidizing at positive potentials. Comparing the cyclic voltammogram of a Cu wire in a TtBACI solution (2 m) with the one obtained in KCI (2 m), it is observed that the corrosion process occurring in KCI is now inhibited in our supramolecular fluid. In its place, a new redox process emerges for the oxidation of Cu(0) to Cu(I) at a potential of ~ -0.52 V (ΔE = Ea-Ec ≈ 1.162 V) and at a lower current density in which Cu(I) is now stabilised against the reduction of the structure forming our supramolecular fluid, see Figure 8. In this process it is also observed that the formation and loss of CuCl₂⁻ (aq) is suppressed as the current at a potential of 0.2 V is now negative, with the decrease of the potential after oxidation.
B) Chronoamperometric experiments were carried out at 0.3 V and the solutions used as electrolytes were analysed. While no change is observed for the aqueous TtBACI solution (2 m) after applying a potential of 0.3 V for 300 seconds, for the potassium solution, which is initially colourless, a colour change to brown is observed in the solution after only 90 seconds, as a result of copper corrosion, which is in agreement with the presence of a band below 300 nm in the ultraviolet-visible spectrum of copper, see Figure 9.
C) RAMAN spectroscopy measurements (in the range of 100 to 1000 cm⁻¹ at a wavelength of 514 nm) of the Cu electrode surface were carried out after chronoamperometric experiments (0.3V), according to example 6B, using TtBACl and KCI as electrolyte at the same concentration (2 m), see Figure 10. Optical images of the analysed areas were also obtained (scale: 30 µm). The electrode surface is completely intact after applying 0.3V for 300 seconds in the presence of TtBACI (only bands related to the presence of the TtBA⁺ cation are observed, *), while in potassium chloride, after only 90 seconds the presence of copper oxide and copper chloride is observed as a consequence of corrosion, which is in agreement with the presence of a blue coating in the analysed images.

### Example 7

Preparation of a Zn-Br button storage cell with the supramolecular fluid of example 1.

### 7.1 Preparation of the supramolecular fluid doped with ZnSO₄ and tetramethyl orthosilicate

3.4 mL of an aqueous solution of ZnSO₄ and 0.1 mL of orthosilicate tetramethyl were added to 1 mL of a 1.8 m aqueous solution of tetrabutylammonium bromide. The mixture was heated at 50 °C under magnetic stirring for 6 hours.

### 7.2. Preparation of the electrodes.

A Zn foil was used as the negative electrode (anode), while modified carbon cloth was used for the positive electrode (cathode). The modified carbon cloth was prepared by mixing commercially available graphite nanoplatelets, Super P carbon black and polyvinyl difluoride (in a mass ratio of 80:10:10), with N-methylpyrrolidone. The homogeneous suspension obtained was deposited on commercial carbon cloth which was dried at 60 °C in a vacuum oven for 12 hours.

### 7.3. Assembly of the button battery.

The electrodes were assembled on standard CR2032 button batteries using electrode discs (15 and 12 mm diameter) and glass fibre filter paper (19 mm diameter). A 15 mm Zn plate was used as the negative electrode and a 12 mm modified carbon cloth as the positive electrode (Figure 11).

### 7.4. Electrochemical tests on the button storage cell

Cyclic voltammetry and electrochemical impedance spectroscopy (EIS) were carried out using a PARSTAT MC 200 potentiostat/galvanostat, while charge and discharge cycle tests were carried out using the CT3002AU electrochemical workstation in the 0.5 to 2 V potential range.

This example shows how to design a button-type storage cell using the fluid from example 1 at a concentration of 1.8 m (Figures 11 and 12). Figure 13 shows the durability of the Zn-Br button storage cell.

Furthermore, the reversibility of the battery has been verified (Figure 14), when there is no supramolecular fluid (Figure 14 b and d), yellow coloured areas can be observed on the surface of the separator (what indicates the presence of tribromide, Br₃⁻), while in the experiment with the supramolecular fluid (Figure 14 a and c) the separator is free from this colouration. The formation of semiclathrates on the electrode (cathode) surface inhibits the secondary reaction of tribromide formation (yellow), allowing the development of electrochemically reversible Zn-Br button storage cells.

The electrochemical stability of the Zn-Br button battery comprising the supramolecular fluid at high potentials was also demonstrated: inhibiting current peaks and the electrolysis of water (Figure 15). As a consequence of an increase in potential, a much more pronounced current peak is observed for the experiment in the absence of supramolecular fluid, compromising the stability and thus the safety of the storage cell. Under the same conditions, it is demonstrated that the Zn-Br button storage cell with the supramolecular fluid is more stable and safer, not only allowing the Br⁻/Br₂ process to be electrochemically reversible, but also inhibiting the electrolysis of water, increasing the potential window by more than 550 mV (i.e. from 2.21 to 2.76 V). Comparing photos 15 e)-j) one can clearly observe the absence of yellow colouration in the separator as opposed to what happens in the experiment in the absence of supramolecular fluid.

## Claims

1. Electrolyte comprising a composition of a quaternary ammonium halide of formula X⁻(Alq₄N)⁺ or a quaternary phosphonium halide of formula X⁻(Alq₄P)⁺, at a concentration equal to or more than 0.6 m
wherein X is selected from chlorine, bromine and iodine, and Alq is a linear or branched C₁-C₄ alkyl.

2. Electrolyte according to claim 1, wherein Alq is butyl.

3. Electrolyte according to claim 1, wherein X is bromine.

4. Battery comprising the electrolyte according to any one of claims 1-3, and at least one electrode.

5. Battery according to claim 4, further comprising an electroactive inorganic salt.

6. Battery according to claim 5, wherein the electroactive inorganic salt is in at least 5% by weight relative to the weight of the electrolyte.

7. Battery according to claims 5-6, wherein the electroactive inorganic salt is selected from a salt of Zinc, Lithium, Sodium, Potassium and Aluminium.

8. Battery according to claims 4-7, wherein the battery is of the button type.

9. Use of the electrolyte according to any one of claims 1-3, in electrochemical reactions or in energy applications.

10. Use of the electrolyte according to any one of claims 1-3, for encapsulating substances.

11. Use, according to claim 10, wherein the encapsulated substances are polar molecules.

12. Use of the electrolyte according to any one of claims 1-3, for inhibiting electrode deterioration.

13. Use according to claim 12, wherein electrode deterioration is due to oxidation or corrosion.
